# EUROPEAN PATENT APPLICATION

(11) **EP 4 534 671 A1**
(43) Date of publication of application: **09.04.2025**
(21) Application number: 23816336.4
(22) Date of filing: 30.05.2023
(51) Int. Cl.: C12N 15/113, A61K 31/7088, A61K 48/00, A61P 25/28

(54) **ANTISENSE OLIGOMERS AGAINST MONOAMINE OXIDASE B AND USE THEREOF**

(30) Priority: 30.05.2022 KR 20220066057
(71) Applicant: Institute for Basic Science, Yuseong-gu Daejeon 34126 (KR); Korea Advanced Institute of Science and Technology, Daejeon 34141 (KR)
(72) Inventor: LEE, C. Justin, Daejeon 34125 (KR); SA, Moonsun, Daejeon 34126 (KR); BHALLA, Mridula Neeraj Kumar, Daejeon 35272 (KR); KIM, Jin Kuk, Daejeon 34141 (KR); JUNG, Eunji, Daejeon 34141 (KR); PARK, Minsung, Daejeon 34141 (KR)
(74) Representative: Mewburn Ellis LLP
(86) International application number: PCT/KR2023/007377
(87) International publication number: WO 2023/234675

(57) **Abstract**

The present invention relates to antisense oligomers against monoamine oxidase B and uses thereof, and more particularly, to antisense oligomers that modulate the expression level of a gene encoding monoamine oxidase B, specifically the mRNA of the gene, or a protein encoded thereby, and uses thereof for preventing, alleviating or treating liver disease, obesity or neurological disease.

## Description

### Technical Field

The present invention relates to antisense oligomers against monoamine oxidase B and uses thereof, and more particularly, to antisense oligomers that modulate the expression level of a gene encoding monoamine oxidase B, specifically the mRNA of the gene, or a protein encoded thereby, and uses thereof for preventing, alleviating or treating liver disease, obesity or neurological disease.

### Background Art

Monoamine oxidase (MAO) is located particularly in the outer membrane of mitochondria in the liver and brain, and is known to promote oxidative deamination of monoamine neurotransmitters such as dopamine. It is known that the reaction promoted by MAO leads to the production of hydrogen peroxide (H₂O₂), which causes oxidative stress and neuronal cell death.

There are two types of MAO: MAOA and MAOB. It is known that MAOB is abundant in neurons and astrocytes that release serotonin and is selectively inhibited by potent inhibitors such as selegiline and rasagiline.

In particular, it is known that the expression level of monoamine oxidase B (MAOB) in the human brain increases with age and the activity thereof increases in neurological diseases (Park et al., Sci Adv. 2019 Mar 20;5(3):eaav0316).

Under this technical background, the inventors of the present application have identified antisense oligomers against monoamine oxidase B, and found that the antisense oligomers may be used for the prevention, alleviation or treatment of neurological disease by regulating the expression of mRNA encoding monoamine oxidase B, thereby completing the present invention.

### Summary of the Invention

An object of the present invention is to provide an antisense oligomer that modulates the expression of a gene encoding monoamine oxidase B.

Another object of the present invention is to provide a pharmaceutical composition for preventing, alleviating or treating liver disease or neurological disease comprising the antisense oligomer. Still another object of the present invention is to provide a method for preventing, alleviating or treating liver disease or neurological disease comprising administering the antisense oligomer. Yet another object of the present invention is to provide a method for preparing a composition for preventing, alleviating or treating liver disease or neurological disease comprising the antisense oligomer.

To achieve the above objects, the present invention provides an oligomer which is capable of hybridizing with at least 8 contiguous nucleobases of the whole pre-mRNA sequence of MAOB and is 10 to 50 nt in length.

The present invention also provides an oligomer which is capable of hybridizing with at least 8 contiguous nucleobases of a sequence selected from the group consisting of SEQ ID NOs: 2, 3, and 9 and is 10 to 50 nt in length.

The present invention also provides an oligomer which is capable of hybridizing with at least 8 contiguous nucleobases of the sequence of SEQ ID NO: 9 and is 10 to 50 nt in length.

The present invention also provides an oligomer comprising a sequence selected from the group consisting of SEQ ID NOs: 22, 23, and 29.

The present invention also provides an oligomer comprising the sequence of SEQ ID NO: 29.

The present invention also provides an oligomer having the following sequence and chemical structure:
T*GAAC*6*6*5*5*5*7*7*6*5*6*ACGA*G;
G*ATCA*6*5*7*7*6*6*8*6*8*6*CAGC*T; or
C*ACTA*5*8*6*5*6*5*8*5*8*8*TAGC*C,
wherein A=2'MOE-A, C=2'MOE-5'-methyl-C, G=2'MOE-G, T=2'MOE-T, 5=DNA-A, 6=DNA-5'-methyl-C, 7=DNA-G, 8=DNA-T, and *=PS, wherein PS represents phosphorothioate, and 2'MOE represents 2'-O-methoxyethyl.

The present invention also provides a composition comprising the oligomer and at least one pharmaceutically acceptable carrier or diluent.

The present invention also provides a composition for preventing, alleviating or treating liver disease or neurological disease comprising the oligomer and at least one pharmaceutically acceptable carrier or diluent. The present invention also provides a method for preventing, alleviating or treating liver disease or neurological disease comprising administering a composition comprising the oligomer and at least one pharmaceutically acceptable carrier or diluent. Another object of the present invention is to provide the use of a composition comprising the oligomer and at least one pharmaceutically acceptable carrier or diluent for the preparation of a composition for preventing, alleviating or treating liver disease or neurological disease.

The present invention also provides a composition for preventing, alleviating or treating fatty liver comprising the oligomer and at least one pharmaceutically acceptable carrier or diluent. The present invention also provides a method for preventing, alleviating or treating fatty liver comprising administering a composition comprising the oligomer and at least one pharmaceutically acceptable carrier or diluent. Another object of the present invention is to provide the use of a composition comprising the oligomer and at least one pharmaceutically acceptable carrier or diluent for the preparation of a composition for preventing, alleviating or treating fatty liver.

The present invention also provides a composition for preventing, alleviating or treating obesity comprising the oligomer and at least one pharmaceutically acceptable carrier or diluent. The present invention also provides a method for preventing, alleviating or treating obesity comprising administering a composition comprising the oligomer and at least one pharmaceutically acceptable carrier or diluent. Another object of the present invention is to provide the use of a composition comprising the oligomer and at least one pharmaceutically acceptable carrier or diluent for the preparation of a composition for preventing, alleviating or treating obesity.

### Brief Description of Drawings

FIGS. 1a and 1b show the results of evaluating the possibility of inhibiting the mRNA expression level of MAOB in the Huh7 cell line by a knockdown (KD) test.
FIG. 2 shows the results of evaluating the inhibition of MAOB expression level by candidates a34, a35 and a41 in the Huh7 cell line using a KD test.
FIG. 3a shows the results of a test performed to evaluate the safety of MAOB antisense oligomers (ASOs) by measurement of survival rate after injection of the ASOs and a test performed to evaluate the inhibition of MAOB expression level.
FIG. 3b shows results indicating that that the expression of MAOB mRNA was inhibited in the cerebrum, thalamus, and cerebellum of the group injected with A41.
FIG. 4a shows the results of a test performed to evaluate the safety of MAOB ASO by measurement of survival rate after injection of the ASO into adult mice and a test performed to evaluate the inhibition of MAOB expression level.
FIG. 4b shows the results of a memory recovery experiment after injection of MAOB ASOs into an Alzheimer's model.
FIG. 5a shows the results of an experiment performed to measure the level of tonic GABA after injection of MAOB ASO into an Alzheimer's model.
FIG. 5b shows the results for the levels of tonic GABA previously measured in the same Alzheimer's animal model.
FIG. 6a shows the results of an experiment performed to evaluate the weight loss effect after injection of MAOB ASO into a fatty liver and obesity animal model.
FIG. 6b shows the results of previous histological observation of changes in triglyceride levels in the livers of the same fatty liver and obesity animal models.

### Detailed Description and Preferred Embodiments of the Invention

Unless otherwise defined, all technical and scientific terms used in the present specification have the same meanings as commonly understood by those skilled in the art to which the present invention pertains. In general, the nomenclature used in the present specification is well known and commonly used in the art.

The antisense oligomer (oligonucleotide) according to the present invention is capable of inhibiting the expression of a gene encoding monoamine oxidase B, specifically mRNA. The antisense oligomer (oligonucleotide) according to the present invention comprises a sequence complementary to the mRNA encoding monoamine oxidase B.

"Antisense activity" means any detectable or measurable activity attributable to the hybridization of an antisense compound to its target nucleic acid. In certain embodiments, antisense activity is a decrease in the amount or expression of a target nucleic acid or protein encoded by such target nucleic acid. The antisense activity according to the present invention acts on a target nucleic acid encoding monoamine oxidase B, thereby reducing the amount or expression of the encoded monoamine oxidase B protein.

"Targeting" or "targeted" means specifically hybridizing to a target nucleic acid and inducing a desired effect.

"Target nucleic acid," "target RNA," and "target RNA transcript" all refer to a nucleic acid capable of being targeted by the antisense oligomer.

The present invention includes an oligomer capable of undergoing hybridization to a target nucleic acid through hydrogen bonding.

"Inhibition" means reduction of target nucleic acid levels or target protein levels in the presence of an antisense compound complementary to a target nucleic acid compared to target nucleic acid levels or target protein levels in the absence of the antisense compound.

"Antisense oligomer" includes a single-stranded oligonucleotide having a nucleobase sequence that permits hybridization to a corresponding region or segment of a target nucleic acid.

The present invention relates to an oligomer which is capable of hybridizing with at least 8 contiguous nucleobases of the whole pre-mRNA sequence of MAOB and is 10 to 50 nt in length.

The above sequence is chrX:43,766,610-43,882,450 (hg38, (-) strand, length 115,841 bp), and may comprise the sequence of SEQ ID NO: 41. The oligomer according to the present invention is capable of hybridizing with at least 8 contiguous nucleobases of the nucleic acid sequence described below.

The oligomer according to the present invention is capable of hybridizing with at least 9, at least 10, at least 11, at least 12, at least 13, at least 14, at least 15, at least 16, at least 17 or at least 18 contiguous nucleobases of the nucleic acid sequence.

In hybridization, the conditions used to achieve a specific level of stringency vary depending on the nature of the nucleic acids being hybridized. For example, the length of the nucleic acids being hybridized, the degree of homology, the nucleotide sequence composition (e.g., GC v. content), and the nucleic acid type (e.g., RNA v. DNA) are considered in selecting hybridization conditions. An additional consideration is whether the nucleic acid is immobilized on, for example, a filter or the like.

Examples of very stringent conditions are as follows: 2×SSC/0.1% SDS at room temperature (hybridization conditions), 0.2×SSC/0.1% SDS at room temperature (low-stringency conditions), 0.2×SSC/0.1% SDS at 42°C (moderate-stringency conditions), and 0.1×SSC at 68°C (high-stringency conditions). The washing process may be performed using any one of these conditions, and, for example, high-stringency conditions or each of the above conditions may be used. The conditions may be applied for 10 to 15 minutes each time in the order described above, or all or some of the conditions described above may be repeatedly applied. As described above, however, the optimal conditions vary depending on the special hybridization reaction involved, and may be determined experimentally. Generally, high-stringency conditions are used for the hybridization of the probe of interest.

At least one of the base pairs involved in hybridization may comprise a G:U, I:A, I:C or I:U wobble pair.

The present invention includes an oligomer which is capable of hybridizing with at least 8 contiguous nucleobases of a sequence selected from the group consisting of SEQ ID NOs: 2, 3, and 9 and is 10 to 50 nt in length.

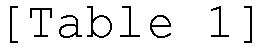

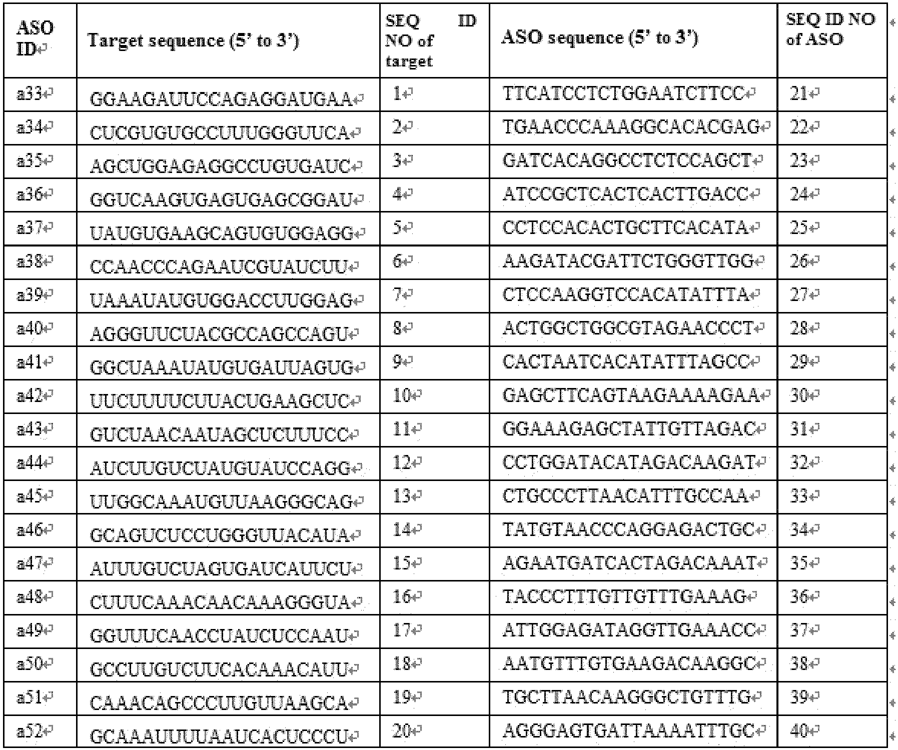

In one embodiment, the present invention includes an antisense oligomer comprising 18 to 21 linked nucleosides, wherein the antisense oligomer is capable of hybridizing with at least 8 contiguous nucleobases of a sequence selected from the group consisting of SEQ ID NOs: 1 to 20 and is 10 to 50 nt in length. The present invention may include an oligomer which is capable of hybridizing with at least 9, at least 10, at least 11, at least 12, at least 13, at least 14, at least 15, at least 16, at least 17 or at least 18 contiguous nucleobases of a sequence selected from the group consisting of SEQ ID NOs: 1 to 20 and is 10 to 50 nt in length.

In one embodiment, the present invention includes an antisense oligomer comprising 15 to 25 linked nucleosides, wherein the oligomer is capable of hybridizing with at least 9, at least 10, at least 11, at least 12, at least 13, at least 14, at least 15, at least 16, at least 17 or at least 18 nucleobases of a sequence selected from the group consisting of SEQ ID NOs: 1 to 20 and is 10 to 50 nt in length.

In one embodiment, the present invention includes an antisense oligomer comprising 18 to 21 linked nucleosides, wherein the oligomer is capable of hybridizing with at least 9, at least 10, at least 11, at least 12, at least 13, at least 14, at least 15, at least 16, at least 17 or at least 18 nucleobases of a sequence selected from the group consisting of SEQ ID NOs: 1 to 20 and is 10 to 50 nt in length.

Specifically, the present invention may include an oligomer which is capable of hybridizing with at least 18, at least 19 or at least 20 contiguous nucleobases of a sequence selected from the group consisting of sequence numbers 1 to 20 and is 10 to 50 nt in length.

The present invention also relates to an oligomer which is capable of hybridizing with at least 8 contiguous nucleobases of a sequence selected from the group consisting of SEQ ID NO: 2, 3 and 9 and is 10 to 50 nt in length.

The present invention may include an oligomer which is capable of hybridizing with at least 9, at least 10, at least 11, at least 12, at least 13, at least 14, at least 15, at least 16, at least 17 or at least 18 contiguous nucleobases of a sequence selected from the group consisting of SEQ ID NOs: 2, 3 and 9 and is 10 to 50 nt in length.

At least one of the base pairs involved in hybridization may comprise a G:U, I:A, I:C or I:U wobble pair.

The present invention also provides an oligomer which is capable of hybridizing with at least 8 contiguous nucleobases of the sequence of SEQ ID NO: 9 and is 10 to 50 nt in length.

The present invention may include an oligomer which is capable of hybridizing with at least 9, at least 10, at least 11, at least 12, at least 13, at least 14, at least 15, at least 16, at least 17 or at least 18 contiguous nucleobases of the sequence of SEQ ID NO: 9 and is 10 to 50 nt in length.

At least one of the base pairs involved in hybridization may comprise a G:U, I:A, I:C or I:U wobble pair.

"Contiguous nucleobases" means nucleobases immediately adjacent to each other. "Internucleoside linkage" refers to the chemical bond between nucleosides. "Linked nucleosides" means adjacent nucleosides which are bonded together.

"Nucleic acid" refers to molecules composed of monomeric nucleotides. Nucleic acids include ribonucleic acids (RNA), deoxyribonucleic acids (DNA), single-stranded nucleic acids, double-stranded nucleic acids, small interfering ribonucleic acids (siRNA), and microRNAs (miRNA). A nucleic acid may also comprise any combination of these elements within a single molecule.

"Nucleobase" means a heterocyclic moiety capable of pairing with a base of another nucleic acid.

"Nucleobase sequence" means the order of contiguous nucleobases independent of any sugar, linkage, or nucleobase modification.

"Nucleoside" means a nucleobase linked to a sugar.

"Nucleotide" means a nucleoside having a phosphate group covalently linked to the sugar portion of the nucleoside.

"Oligomer" means a polymer of linked monomeric subunits which is capable of hybridizing to at least a region of a nucleic acid molecule.

"Oligonucleotide" means a polymer of linked nucleosides each of which can be modified or unmodified, independently of one another.

"Single-stranded oligonucleotide" means an oligonucleotide which is not hybridized to a complementary strand.

At least one of base pairs involved in hybridization may comprise a G:U, I:A, I:C or I:U wobble pair. The antisense oligomer according to the present invention may comprise, for example, a sequence having at least 90% sequence homology with a sequence selected from the group consisting of SEQ ID NOs: 21 to 40.

Specifically, the antisense oligomer may comprise a sequence having at least 91%, at least 92%, at least 93%, at least 94%, at least 95%, at least 96%, at least 97%, at least 98%, at least 99% or 100% sequence homology with a sequence selected from the group consisting of SEQ ID NOs: 21 to 40.

The antisense oligomer according to the present invention may comprise, for example, a sequence having at least 90% sequence homology with at least one sequence selected from the group consisting of SEQ ID NOs: 22, 23 and 29.

Specifically, the antisense oligomer may comprise a sequence having at least 91%, at least 92%, at least 93%, at least 94%, at least 95%, at least 96%, at least 97%, at least 98%, at least 99% or 100% sequence homology with at least one sequence selected from the group consisting of SEQ ID NOs: 22, 23 and 29. The antisense oligomer according to the present invention may comprise a sequence selected from the group consisting of SEQ ID NOs: 22, 23 and 29.

The antisense oligomer according to the present invention may comprise, for example, a sequence having at least 90% sequence homology with the sequence of SEQ ID NO: 29.

Specifically, the antisense oligomer may comprise a sequence having at least 91%, at least 92%, at least 93%, at least 94%, at least 95%, at least 96%, at least 97%, at least 98%, at least 99% or 100% sequence homology with the sequence of SEQ ID NO: 29. The antisense oligomer according to the present invention may comprise, for example, the sequence of SEQ ID NO: 29.

As used herein, the term "homology" means the percentage of identity between two polynucleotide moieties. "Identity" means the extent to which sequences are determined to be functionally or structurally identical based on polynucleotide sequences over a comparison window. Sequence homology may be determined by comparing sequences using standard software, for example, a program called BLASTN or BLASTX, which has been developed based on BLAST (Proc. Natl. Acad. Sci. USA, 90, 5873-5877, 1993).

In one embodiment, the antisense oligomer according to the present invention may comprise 20 linked nucleosides. Specifically, the antisense oligomer according to the present invention may comprise at least one sequence selected from the group consisting of SEQ ID NOs: 22, 23 and 29.
TGAACCCAAAGGCACACGAG (SEQ ID NO: 22);
GATCACAGGCCTCTCCAGCT (SEQ ID NO: 23); and
CACTAATCACATATTTAGCC (SEQ ID NO: 29).

In particular, the antisense oligomer according to the present invention may comprise the sequence of SEQ ID NO: 29.

In this specification, the complementary sequence is meant to encompass some base deletions and incomplete complementarity corresponding to the extent of being able to inhibit the expression of an mRNA encoding monoamine oxidase B.

"Nucleoside" means a nucleobase linked to a sugar, and "nucleotide" means a nucleoside having a phosphate group covalently linked to the sugar portion of the nucleoside. "Nucleobase" means a heterocyclic moiety capable of pairing with a base of another nucleic acid.

"Oligomer" means a polymer of linked monomeric subunits which is capable of hybridizing to at least a region of a nucleic acid molecule.

The nucleoside may comprise a modification. For example, the nucleoside may comprise a modified internucleoside linkage, or may comprise a modified sugar.

Specifically, the internucleoside linkage may be a phosphorothioate, boranophosphate, or methyl phosphonate linkage.

The internucleoside linkage according to the present invention may be a linkage of sooosssssssssssooos, wherein s may be a phosphorothioate internucleoside linkage, and o may be a phosphodiester internucleoside linkage.

The nucleoside may comprise the following modified sugar:
Modification by substitution with 2'-O-methyl (2'-O-Me), 2'-O-methoxyethyl (2'MOE), 2'-O-methoxyethyl-5'methyl, 2'-O-aminoethyl, 5'-methyl, 2'-O-propyl, 2'-methylthioethyl, or 2'-fluoro at the 2' carbon position of the sugar structure.

The nucleoside may comprise, for example, a phosphorothioate, boranophosphate, or methyl phosphonate internucleoside linkage; and modification by substitution with 2'-O-methyl (2'-O-Me), 2'-O-methoxyethyl (2'MOE), 2'-O-methoxyethyl-5'methyl, 2'-O-aminoethyl, 5'-methyl, 2'-O-propyl, 2'-methylthioethyl, or 2'-fluoro at the 2' carbon position of the sugar structure.

The nucleoside may comprise, for example, a modification selected from the group consisting of the following modifications: The modifications may include modifications of the sugar moiety, for example, modifications at the 2' carbon position of the sugar structure in the nucleotide, specifically modifications with 2'-O-methyl (2'-O-Me), 2'-O-methoxyethyl (2'MOE), 2'-O-methoxyethyl-5'methyl, 2'-O-aminoethyl, 5'-methyl, 2'-O-propyl, 2'-methylthioethyl, or 2'-fluoro; phosphorothioate, boranophosphate, or methyl phosphonate internucleoside linkage modifications; modifications to the form of peptide nucleic acid (PNA), locked nucleic acid (LNA), or unlocked nucleic acid (UNA); and a phosphate group.

"2'-O-methoxyethyl" (also 2'-MOE or 2'-O(CH₂)₂-OCH₃) refers to an O-methoxy-ethyl modification at the 2' position of a furosyl ring.

"2'-O-methoxyethyl nucleotide" means a nucleotide comprising a 2'-O-methoxyethyl modified sugar moiety.

"Modified sugar" refers to a substitution or change from a natural sugar.

"5-methylcytosine" means a cytosine modified with a methyl group attached to the 5' position.

"Modified internucleoside linkage" refers to a substitution or any change from a naturally occurring internucleoside bond.

"Modified nucleobase" refers to any nucleobase other than adenine, cytosine, guanine, thymidine, or uracil. "Unmodified nucleobase" means the purine bases adenine (A) and guanine (G), and the pyrimidine bases thymine (T), cytosine (C), and uracil (U).

"Modified nucleotide" means a nucleotide having, independently, a modified sugar moiety, modified internucleoside linkage, or modified nucleobase. "Modified nucleoside" means a nucleoside having, independently, a modified sugar moiety or modified nucleobase.

"Modified oligonucleotide" means an oligomer comprising at least one modified nucleotide.

The antisense oligomer according to the present invention may comprise trivalent N-acetylgalactosamine (GalNAc) or pentavalent N-acetylgalactosamine (GalNAc), without being limited thereto.

The antisense oligomer according to the present invention may comprise a 2'-O-methoxyethyl (2'MOE), 2'-O-methoxyethyl-5'methyl, 5'-methyl or phosphorothioate linkage modification.

In another aspect, the present invention relates to an antisense oligomer comprising 12 to 30 linked nucleosides, wherein the oligomer comprises at least 8 contiguous nucleobases of a sequence selected from the group consisting of SEQ ID NOs: 2, 3 and 9.

In one embodiment, the antisense oligomer comprises an antisense oligomer comprising 18 to 21 linked nucleosides, wherein the antisense oligomer comprises at least 8 contiguous nucleobases of a sequence selected from the group consisting of SEQ ID NOs: 2, 3 and 9. The antisense oligomer may comprise at least 9, at least 10, at least 11, at least 12, at least 13, at least 14 or at least 15 contiguous nucleobases of a sequence selected from the group consisting of SEQ ID NOs: 2, 3 and 9.

In one embodiment, the antisense oligomer may be an antisense oligomer comprising 15 to 25 linked nucleosides, wherein the antisense oligomer may comprise at least 9, at least 10, at least 11, at least 12, at least 13, at least 14 or at least 15 contiguous nucleobases of a sequence selected from the group consisting of SEQ ID NOs: 2, 3 and 9.

In one embodiment, the antisense oligomer may be an antisense oligomer comprising 18 to 21 linked nucleosides, wherein the antisense oligomer may comprise at least 9, at least 10, at least 11, at least 12, at least 13, at least 14 or at least 15 contiguous nucleobases of a sequence selected from the group consisting of SEQ ID NOs: 2, 3 and 9.

Specifically, the antisense oligomer may comprise at least 18, at least 19, or at least 20 contiguous nucleobases selected from the group consisting of sequence numbers 2, 3, and 9.

In another aspect, the present invention relates to an antisense oligomer comprising 12 to 30 linked nucleosides, wherein the antisense oligomer comprise at least 8 contiguous nucleobases of the sequence of SEQ ID NO: 9.

In one embodiment, the antisense oligomer is an antisense oligomer comprising 18 to 21 linked nucleosides, wherein the antisense oligomer comprises at least 8 contiguous nucleobases of a sequence of SEQ ID NO: 9. The antisense oligomer may comprise at least 9, at least 10, at least 11, at least 12, at least 13, at least 14 or at least 15 contiguous nucleobases of a sequence selected from the group consisting of SEQ ID NO: 2, 3 and 9.

In one embodiment, the antisense oligomer may be an antisense oligomer comprising 15 to 25 linked nucleosides, wherein the antisense oligomer may comprise at least 9, at least 10, at least 11, at least 12, at least 13, at least 14 or at least 15 contiguous nucleobases of the sequence of SEQ ID NO: 9.

In one embodiment, the antisense oligomer may be an antisense oligomer comprising 18 to 21 linked nucleosides, wherein the antisense oligomer may comprise at least 9, at least 10, at least 11, at least 12, at least 13, at least 14 or at least 15 contiguous nucleobases of the sequence of SEQ ID NO: 9.

Specifically, the antisense oligomer may comprise at least 18, at least 19 or at least 20 contiguous nucleobases of the sequence of SEQ ID NO: 9.

The present invention relates to an antisense oligomer that targets a gene encoding monoamine oxidase B, the antisense oligomer comprising a sequence selected from the group consisting of SEQ ID NO: 2, 3 and 9. In particular, the antisense oligomer according to the present invention may comprise the sequence of SEQ ID NO: 9.

The oligomer according to the present invention may be 13 to 35 nt in length or 16 to 25 nt in length. The oligomer may be 16 nt, 17 nt, 18 nt, 19 nt, 20 nt, 21 nt, 22 nt, 23 nt, 24 nt or 25 nt in length.

Each of the nucleosides of a sequence selected from the group consisting of SEQ ID NOs: 2, 3 and 9 may comprise a modification. For example, the nucleotide may comprise a modified internucleoside linkage or may comprise a modified sugar.

Specifically, the internucleoside linkage may be a phosphorothioate, boranophosphate, or methyl phosphonate linkage. At least one of the modified internucleoside linkages may be a phosphorothioate linkage.

The internucleoside linkage according to the present invention may be a linkage of sooosssssssssssooos, wherein s may be a phosphorothioate internucleoside linkage, and o may be a phosphodiester internucleoside linkage.

At least one nucleoside of the oligomer may comprise a modified sugar different from DNA or RNA. The nucleoside may comprise the following modified sugar:

Modification by substitution with 2'-O-methyl (2'-O-Me), 2'-O-methoxyethyl (2'MOE), 2'-O-methoxyethyl-5'methyl, 2'-O-aminoethyl, 5'-methyl, 2'-O-propyl, 2'-methylthioethyl, or 2'-fluoro at the 2' carbon position of the sugar structure.

The nucleoside may comprise, for example, a modification selected from the group consisting of the following modifications: The modifications may include modifications of the sugar moiety, for example, modifications at the 2' carbon position of the sugar structure in the nucleotide, specifically modifications with 2'-O-methyl (2'-O-Me), 2'-O-methoxyethyl (2'MOE), 2'-O-methoxyethyl-5'methyl, 2'-O-aminoethyl, 5'-methyl, 2'-O-propyl, 2'-methylthioethyl, or 2'-fluoro; phosphorothioate, boranophosphate, or methyl phosphonate internucleoside linkage modifications; modifications to the form of peptide nucleic acid (PNA), locked nucleic acid (LNA), or unlocked nucleic acid (UNA); and a phosphate group.

The antisense oligomer according to the present invention may comprise a modification with a 2'-O-methoxyethyl (2'MOE), 2'-O-methoxyethyl-5'methyl, 5'-methyl or phosphorothioate linkage. At least one nucleoside of the oligonucleotide may have 2'-O-methoxyethyl (2'MOE).

Each of the nucleosides of the sequence of SEQ ID NO: 9 may comprise a modification. For example, the nucleotide may comprise a modified internucleoside linkage or may comprise a modified sugar.

Specifically, the internucleoside linkage may be a phosphorothioate, boranophosphate, or methyl phosphonate linkage.

The internucleoside linkage according to the present invention may be a linkage of sooosssssssssssooos, wherein s may be a phosphorothioate internucleoside linkage, and o may be a phosphodiester internucleoside linkage.

The nucleoside may comprise the following modified sugar:
Modification by substitution with 2'-O-methyl (2'-O-Me), 2'-O-methoxyethyl (2'MOE), 2'-O-methoxyethyl-5'methyl, 2'-O-aminoethyl, 5'-methyl, 2'-O-propyl, 2'-methylthioethyl, or 2'-fluoro at the 2' carbon position of the sugar structure.

The nucleoside may comprise, for example, a modification selected from the group consisting of the following: modifications of the sugar moiety, for example, modifications at the 2' carbon position of the sugar structure in the nucleotide, specifically modifications with 2'-O-methyl (2'-O-Me), 2'-O-methoxyethyl (2'MOE), 2'-O-methoxyethyl-5'methyl, 2'-O-aminoethyl, 5'-methyl, 2'-O-propyl, 2'-methylthioethyl, or 2'-fluoro; phosphorothioate, boranophosphate, or methyl phosphonate internucleoside linkage modifications; modifications to the form of peptide nucleic acid (PNA), locked nucleic acid (LNA), or unlocked nucleic acid (UNA); and a phosphate group.

The antisense oligomer according to the present invention may comprise a modification with a 2'-O-methoxyethyl (2'MOE), 2'-O-methoxyethyl-5'methyl, 5'-methyl or phosphorothioate linkage.

At least one nucleoside of the oligomer may be a nucleotide analog having an additional ring formed in the sugar moiety.

The modified sugar of at least one nucleoside of the above oligomer may be a bicyclic sugar moiety (cEt) or locked nucleic acid (LNA).

"Bicyclic sugar" means a furosyl ring modified by the bridging of two non-geminal ring atoms. A bicyclic sugar is a modified sugar.

The bicyclic sugar moiety comprises a 4'-CH(CH₃)-O-2' bridge. In one embodiment, at least one modified sugar comprises 2'-O-methoxyethyl.

The oligomer may be an oligomer that a. comprises: a gap segment consisting of 8 to 12 linked deoxynucleosides;
a 5' wing segment consisting of 3 to 7 linked nucleosides; and
a 3' wing segment consisting of 3 to 7 linked nucleosides;
b. wherein the gap segment is positioned between the 5' wing segment and the 3' wing segment, and
c. wherein each nucleoside of each wing segment comprises a modified sugar.

The oligomer may be an oligomer which is 20 nucleosides in length and comprises: a gap segment consisting of 10 linked deoxynucleosides;
a 5' wing segment consisting of 5 linked nucleosides; and
a 3' wing segment consisting of 5 linked nucleosides;
wherein the gap segment is positioned between the 5' wing segment and the 3' wing segment, and
wherein each nucleoside of each wing segment comprises 2'-O-methoxyethyl ribose (2'-MOE ribose).

The internucleoside linkage in the oligomer may be a linkage of sooosssssssssssooos linkage, wherein s may be a phosphorothioate internucleoside linkage, and o may be a phosphodiester internucleoside linkage.

The oligomer may be an oligomer which is 18 nucleosides in length and comprises:
a gap segment consisting of 8 linked deoxynucleosides;
a 5' wing segment consisting of 5 linked nucleosides; and
a 3' wing segment consisting of 5 linked nucleosides;
wherein the gap segment is positioned between the 5' wing segment and the 3' wing segment, and
wherein each nucleoside of each wing segment comprises 2'-O-methoxyethyl ribose (2'-MOE ribose).

The oligomer may be an oligomer which is 20 nucleosides in length and comprises:
a gap segment consisting of 8 linked deoxynucleosides;
a 5' wing segment consisting of 6 linked nucleosides; and
a 3' wing segment consisting of 6 linked nucleosides;
wherein the gap segment is positioned between the 5' wing segment and the 3' wing segment, and
wherein each nucleoside of each wing segment comprises 2'-O-methoxyethyl ribose (2'-MOE ribose).

"Gapmer" means a chimeric antisense compound in which an internal region having a plurality of nucleosides that support RNase H cleavage is positioned between external regions having one or more nucleosides, wherein the nucleosides comprising the internal region are chemically distinct from the nucleoside or nucleosides comprising the external regions. The internal region may be referred to as a "gap segment" and the external regions may be referred to as "wing segments."

"Gap-widened" means a chimeric antisense compound having a gap segment of 12 or more contiguous 2'-deoxynucleosides positioned between and immediately adjacent to 5' and 3' wing segments having from 1 to 6 nucleosides.

In one embodiment, at least one nucleoside may comprise a modified nucleobase. The modified nucleobase may be 5-methylcytosine (5mC).

Specifically, the antisense oligomer according to the present invention may be selected from the group consisting of those shown below:

| **ID** | **Sequence** | **Type** | **Chemistry** | **Target gene** | **Species** |
|---|---|---|---|---|---|
| a34 | TGAACCCAAAGGCACACGAG | gapmer | T*GAAC*6*6*5*5*5*7*7*6*5*6*ACGA*G | MAOB | human |
| a35 | GATCACAGGCCTCTCCAGCT | gapmer | G*ATCA*6*5*7*7*6*6*8*6*8*6*CAGC*T | MAOB | human |
| a41 | CACTAATCACATATTTAGCC | gapmer | C*ACTA*5*8*6*5*6*5*8*5*8*8*TAGC*C | MAOB | human |

PS, phosphorothioate; 2'MOE, 2'-O-methoxyethyl. A=2'MOE-A, C=2'MOE-5'-methyl-C, G=2'MOE-G, T=2'MOE-T, 5=DNA-A, 6=DNA-5'-methyl-C, 7=DNA-G, 8=DNA-T, *=PS

The present invention also relates to a pharmaceutical composition for preventing, alleviating or treating neurological disease comprising the oligomer. The present invention also relates to a method for preventing, alleviating or treating neurological disease comprising a step of administering the antisense oligomer to a subject. The present invention also relates to the use of the oligomer for preparation of a composition for preventing, alleviating or treating a neurological disease comprising the antisense oligomer.

The oligomer according to the present invention may be an MAOB inhibitor. The present invention relates to a method for reducing the amount of MAOB mRNA or protein in an animal, comprising administering to the animal the oligomer according to the present invention to reduce the amount of the MAOB mRNA or protein. This can reduce the formation of oxygen radicals and increase the amounts of useful monoamines in the brain. In addition, in brain diseases and brain injuries, including Alzheimer's disease, MAOB promotes putrescine metabolism in reactive astrocytes, causing excessive production of GABA. Therefore, the antisense oligomer MAOB inhibitor according to the present invention can restore neuronal signal transmission and brain function by acting as an inhibitor of GABA production in astrocytes. The neurological disease may be, for example, Parkinson's disease (PD), Alzheimer's disease (AD), Huntington's disease (HD), amyotrophic lateral sclerosis (ALS), frontotemporal dementia, cortico-basal degeneration, or progressive supranuclear palsy (PSP), without being limited thereto.

The oligomer according to the present invention may be used for the prevention, alleviation or treatment of liver disease. The liver disease may specifically be fatty liver. The present invention also relates to a pharmaceutical composition for preventing, alleviating or treating liver disease comprising the oligomer. The present invention also relates to a method for preventing, alleviating or treating liver disease comprising a step of administering the antisense oligomer to a subject. The present invention also relates to the use of the oligomer for preparation of a composition for preventing, alleviating or treating liver disease comprising the antisense oligomer.

The present invention also relates to a pharmaceutical composition for preventing, alleviating or treating obesity comprising the oligomer. The present invention also relates to a method for preventing, alleviating or treating obesity comprising a step of administering the antisense oligomer to a subject. The present invention also relates to the use of the oligomer for preparation of a composition for preventing, alleviating or treating obesity comprising the antisense oligomer.

In the present specification, the term "treatment" may be meant to include alleviation or improvement of symptoms, reduction of the scope of a disease, delay or alleviation of the progress of a disease, improvement, alleviation or stabilization of a disease condition, partial or complete recovery, extension of survival, and other therapeutically favorable results.

The present invention relates to a composition comprising the oligomer and at least one pharmaceutically acceptable carrier or diluent.

The pharmaceutical composition may comprise at least one pharmaceutically acceptable carrier in addition to the active ingredient. The pharmaceutically acceptable carrier must be compatible with the active ingredient of the present invention, and may be one or a mixture of two or more selected from among saline solution, sterile water, Ringer's solution, buffered saline, dextrose solution, maltodextrin solution, glycerol, and ethanol. The pharmaceutical composition may, if necessary, comprise other conventional additives such as antioxidants, buffers, and bacteriostatic agents. In addition, the pharmaceutical composition may be formulated into injectable formulations such as aqueous solutions, suspensions, emulsions, etc. by additionally adding a diluent, a dispersant, a surfactant, a binder, and a lubricant.

The pharmaceutical composition encompasses any pharmaceutically acceptable salt, ester, salt of such ester, or any other oligomer, which upon administration to an animal, including a human, is capable of providing, either directly or indirectly, a biologically active metabolite or residue thereof. Accordingly, for example, the present invention also relates to pharmaceutically acceptable salts of the antisense oligomer, prodrugs, pharmaceutically acceptable salts of such prodrugs, and other bioequivalents. Suitable pharmaceutically acceptable salts include, but are not limited to, sodium and potassium salts.

The composition may be co-administered with a second agent may be co-administered. When the second agent is included in the "co-administration", it may be a single pharmaceutical composition or a separate pharmaceutical composition. The second agent may be administered via a route that is the same as or different from the route of administration of the composition. Co-administration includes simultaneous or sequential administration.

The present invention also relates to a method for reducing the amount of MAOB mRNA or protein in an animal, comprising administering to the animal a composition according to the present invention to reduce the amount of the MAOB mRNA or protein.

The animal may be, but is not limited to, a human, a primate such as a monkey, a dog, a pig, a cow, a sheep, a goat, a mouse, or a rat. The animal may be, for example, a human.

The method of administration of the pharmaceutical composition may be determined by a person skilled in the art based on the symptoms and severity of the patient's disease.

The pharmaceutical composition of the present invention may be administered parenterally. The composition according to the present invention may be administered, for example, via an oral, intravenous, intramuscular, intraarterial, intramedullar, intrathecal, intracardiac, transdermal, subcutaneous, intraperitoneal, enteral, sublingual, or topical route, without being limited thereto. The pharmaceutical composition of the present invention may be administered to the central nervous system (CNS). The pharmaceutical composition of the present invention may be administered by intrathecal injection.

In some cases, the pharmaceutical composition of the present invention may be administered by intravenous injection or subcutaneous injection.

The dose of the composition according to the present invention varies depending on the patient's weight, age, sex, health condition, diet, administration time, mode, excretion rate, or disease severity, and may be easily determined by a person skilled in the art. In addition, the composition of the present invention may be formulated into a suitable dosage form using a known technique for clinical administration.

"Dose" means a specified quantity of a pharmaceutical agent provided in a single administration or over a specified amount of time. In certain embodiments, a dose may be administered in two or more boluses, tablets, or injections. For example, in certain embodiments, where subcutaneous administration is desired, the desired dose requires a volume not easily accommodated by a single injection, and two or more injections may be used to achieve the desired dose. The pharmaceutical agent may be administered by infusion over an extended period of time or continuously. The dose may be described as the amount of the pharmaceutical agent per hour, day, week, or month.

### Examples

Hereinafter, the present invention will be described in more detail by way of examples. These examples are only intended to illustrate the present invention, and it will be apparent to those skilled in the art that the scope of the present invention is not to be construed as being limited by these examples.

### Example 1. MAOB Knockdown ASO Screening

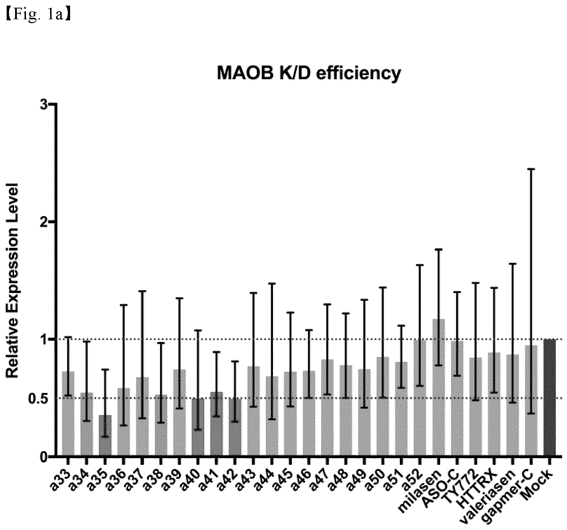

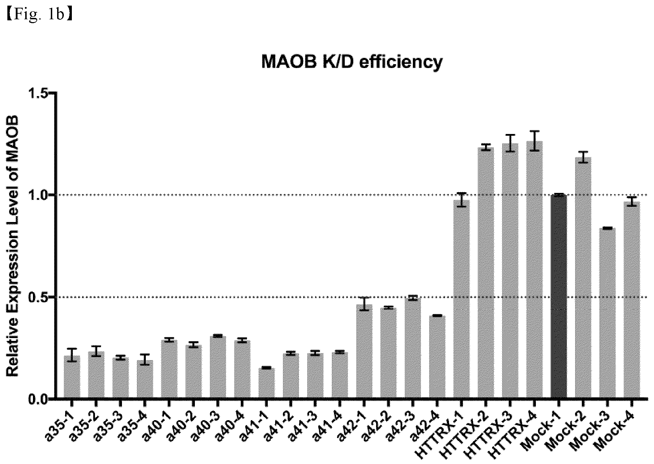

To evaluate the possibility of inhibiting the expression level of MAOB mRNA in the Huh7 cell line, a test was performed. 200 nM of each of ASO candidates a33 to a52 was transfected for 48 hours. Referring to FIGS. 1a and 1b, among the candidates a33 to a52, the candidates a34, a35 and a41 significantly inhibited the expression level of MAOB (error bar: 95% confidence interval).

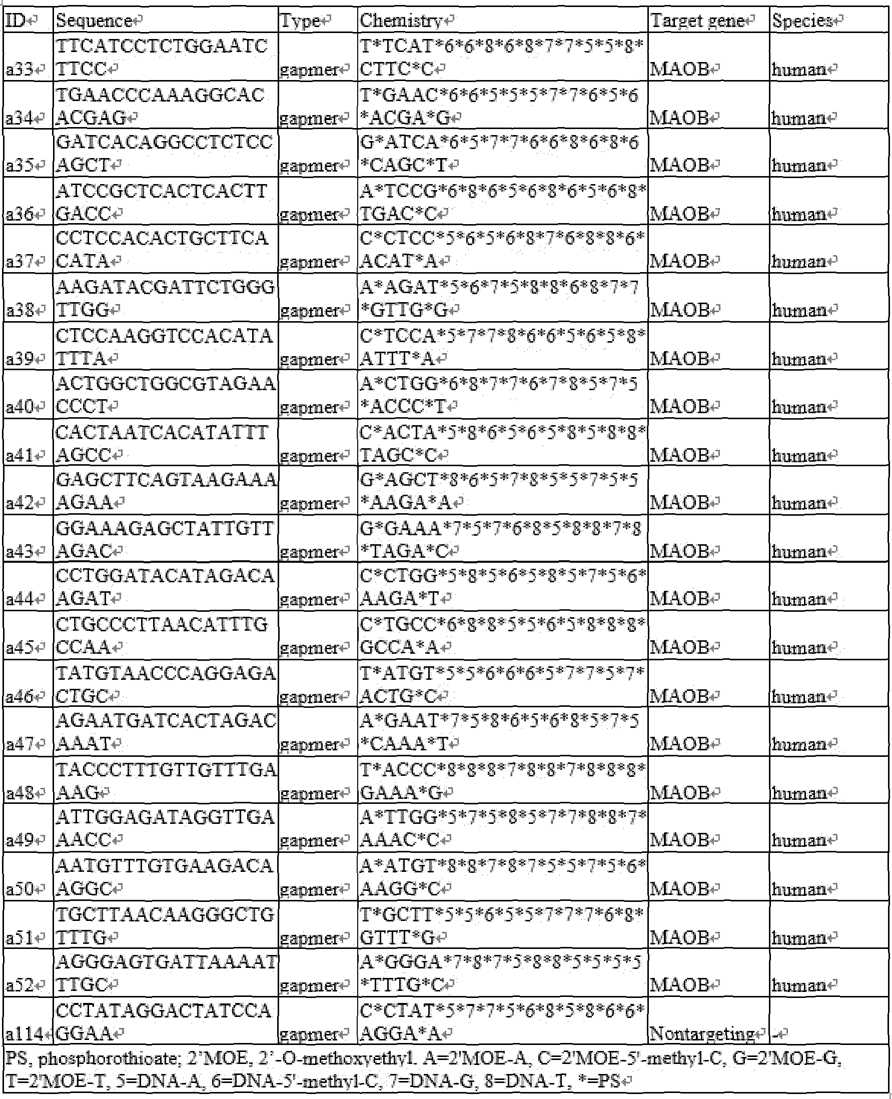

### Example 2. MAOB Knockdown Lead ASO Test

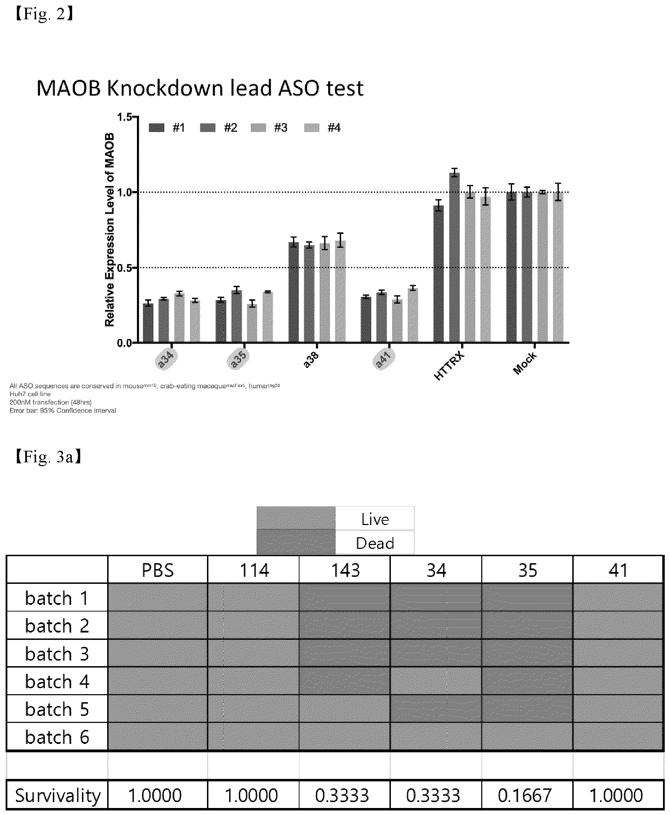

A knockdown (KD) test was performed in the Huh7 cell line. 200 nM of each ASO was transfected for 48 hours. The candidates a34, a35 and a41 selected by screening from the candidates a33 to a52 were repeatedly tested in the Huh7 cell line, and as a result, as shown in FIG. 2, they showed repetitive and effective inhibition of the expression level of MAOB.

### Example 3. MAOB ASO KD Test

### 3-1. MAOB ASO KD Test in p1 Mice

25 µg of each of PBS, control ASO (a114), and MAOB-targeting ASO (a41) was injected intracerebroventricularly (ICV injection, 2.0 mm ventral from the skin surface) into 1-day-old C57BL/J mice. A 33G injection needle was used for ICV injection. Eight days after ICV injection, all brains were dissected into cerebrum, thalamus, and cerebellum. The RNeasy mini prep kit (Qiagen) was used to isolate and purify mRNA from the ASO-injected mice. The SuperScript III First-Strand Synthesis System (Invitrogen^{™}) was used for cDNA synthesis. qRT-PCR was performed using PowerUp^{™} SYBR^{™} Green Master Mix (Applied Biosystems^{™}), 500 nM primers, and 10 ng cDNA from the ASO-injected brain. The following primer sequences were used for qRT-PCR: mMAOB forward: 5-'AGTTGAGCGGCTGATACACT-3', mMAOB reverse: 5'-TGGCCCATCTCATCCATTGT-3'; GAPDH forward: 5'-TGATGACATCAAGAAGGTGGTGAAG-3', GAPDH reverse: 5'-TCCTTGGAGGCCATGTAGGCCAT-3'. Relative mRNA expression levels were calculated using the comparative Ct method and normalized to GAPDH mRNA levels.

Referring to FIG. 3a, the results of the safety evaluation test performed by measurement of survival rate after of MAOB ASOs and the test performed to evaluate the inhibition of MAOB expression level (1-day-old) showed that, among the ASOs candidates a34, a35 and a41 injected into 1-day-old C57BL/6j mice, the candidate a41 had no problem with survival of the mice.

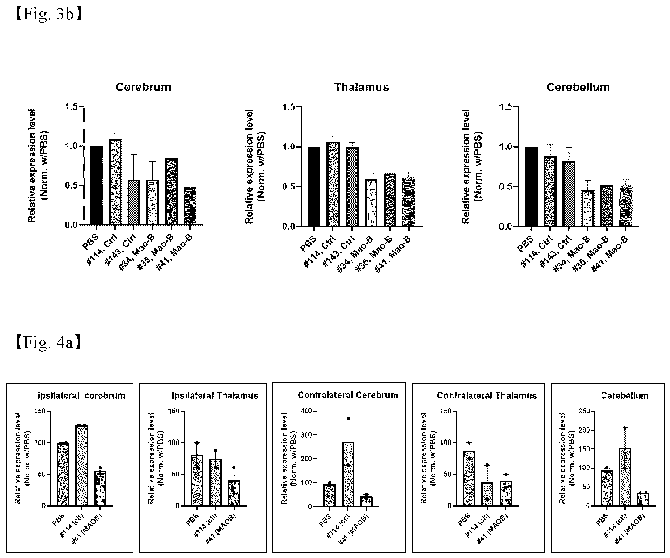

Referring to FIG. 3b, as a result of injecting the ASO candidates a34, a35 and a41 into one-day-old C57BL/6j mice and measuring the expression level of MAOB mRNA, it was confirmed that the expression of MAOB mRNA was inhibited in the cerebrum, thalamus, and cerebellum of the group injected with a41.

### 3-2. MAOB ASO KD Test in APP/PS1 Mice

500 µg of each of control ASO (a114) and MAOB-targeting ASO (a41) was injected intracerebroventricularly into adult C57BL/6J mice (toxicity and knockdown efficacy testing) and APP/PS1 mice. The mice were anesthetized with vaporized isoflurane and placed in a stereotaxic frame (Kopf). The scalp was incised and a hole was drilled in the skull of the ventricle (anterior/posterior +0.3 mm, medial/external - 0.8 mm from bregma, dorsal/ventral -2.5 mm from brain surface). ASO (at a concentration of 100 µg/ µL) was loaded into a glass needle and infused into the ventricle at a rate of 1 µl/min for 5 minutes (a total of 5 µl) using a syringe pump (KD Scientific). Adult C57BL/6J mice were used for knockdown efficiency and toxicity tests. The brains were dissected 2 weeks after injection into the thalamus, cerebrum, and cerebellum. RNA was isolated using the RNeasy miniprep kit (Qiagen). cDNA was synthesized from mRNA using oligodT primers and the SuperScript III First-Strand Synthesis System (Invitrogen) kit. Gene expression levels were measured by qRT-PCR of 10 ng cDNA using the PowerUp SYBR Green Master Mix (Applied Biosystems). APP/PS1 mice were used for behavioral testing and electrophysiology 2 weeks or more after injection.

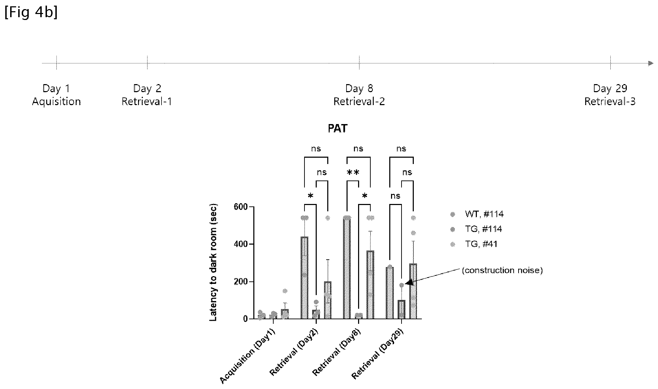

FIG. 4a shows the results of the test performed to evaluate the safety of MAOB ASO by measurement of survival rate after injection of the ASO into adult mice and the test performed to evaluate the inhibition of MAOB expression level. As a result of injecting the ASO candidate a41 into 12-month-old C57BL/6j mice and measuring the expression level of MAOB mRNA, it was confirmed that the expression of MAOB mRNA was inhibited in the cerebrum, thalamus, and cerebellum.

Before the passive avoidance test, mice were handled daily by the experimenter's hand for 7 days. In the passive avoidance test, mice were placed in two-compartment shuttle chambers (light/dark) with a constant-current shock generator (Scitech). On the acquisition day, the mouse was placed in the corner of the light chamber for 60 seconds (familiarization), and the shutter between the two chambers was opened. When the mouse passed through the shutter, the shutter was immediately closed and an aversive electric shock (0.5 mA, 2 seconds) was delivered to the grid floor. After electric shock, the mouse was allowed to return to the cage, and the holding test was performed 24 h after the acquisition test. For the holding test, the mouse was placed in the corner of the light chamber, and the shutter was opened after 1 min. The latency to enter the dark chamber was automatically recorded up to 540 seconds.

FIG. 4a shows the results of the memory recovery experiment after injection of MAOB ASO into the Alzheimer's model. As a result of injecting MAOB ASO a41 into the Alzheimer's animal model APP/PS1, it was confirmed that the reduced memory was restored to the level similar to that of the normal group.

### 3-3. MAOB ASO KD Test (Tonic GABA Recording) in APP/PS1 Mice

Brain slices were prepared from APP/PS1 transgenic mice and WT littermates aged approximately 13 to 16 months. Mice were deeply anesthetized with isoflurane. After anesthesia, the brain was rapidly excised from the skull and submerged in ice-cold NMDG-based cutting solution containing 93 mM NMDG, 2.5 mM KCl, 1.2 mM NaH₂PO₄, 30 mM NaHCO₃, 20 mM HEPES, 25 mM glucose, 5 mM sodium ascorbate, 2 mM thiourea, 3 mM sodium pyruvate, 10 mM MgSO₄, 0.5 mM CaCl₂ (300 to 310 mOsm, pH adjusted with 10 N HCl).

The cutting solution was gassed with 95% O₂ and 5% CO₂. Hippocampal slices were obtained by vibrating microtome (DSK, Linearslicer 7N), and 300-µm-thick coronal hippocampal slices were maintained at room temperature in a submerged chamber containing extracellular artificial cerebrospinal fluid solution.

Liquid (aCSF) solution (126 mM NaCl, 24 mM NaHCO₃, 1 mM NaH₂PO₄, 2.5 mM KCl, 2.5 mM CaCl₂, 2 mM MgCl₂, and 10 mM D- (+) -glucose (pH 7.4)). aCSF solution was gassed with 95% O₂ and 5% CO₂. Slices were incubated in aCSF at room temperature for at least 1 h before recording. Whole-cell patch clamp recordings were made from granule cell somata located in the DG. The holding potential was -70 mV. The pipette resistance was typically 5 to 7 MΩ and the pipette was filled with an internal solution (135 mM CsCl, 4 mM NaCl, 0.5 mM CaCl₂, 10 mM HEPES, 5 mM EGTA, 2 mM Mg-adenosine triphosphate, 0.5 mM Na₂-guanosine triphosphate, and 10 mM QX-314 (pH adjusted to 7.2 with CsOH) (278 to 285 mOsm). Before measuring tonic current, baseline current was stabilized with d-AP5 (50 mM) and 6-cyano-7-nitroquinoxaline-2,3-dione (CNQX, 20 µM). The amplitude of tonic GABA current was measured by the baseline shift after bicuculline (100 mM) administration using the Clampfit program. Tonic current was measured from baseline to the bicuculline treatment current.

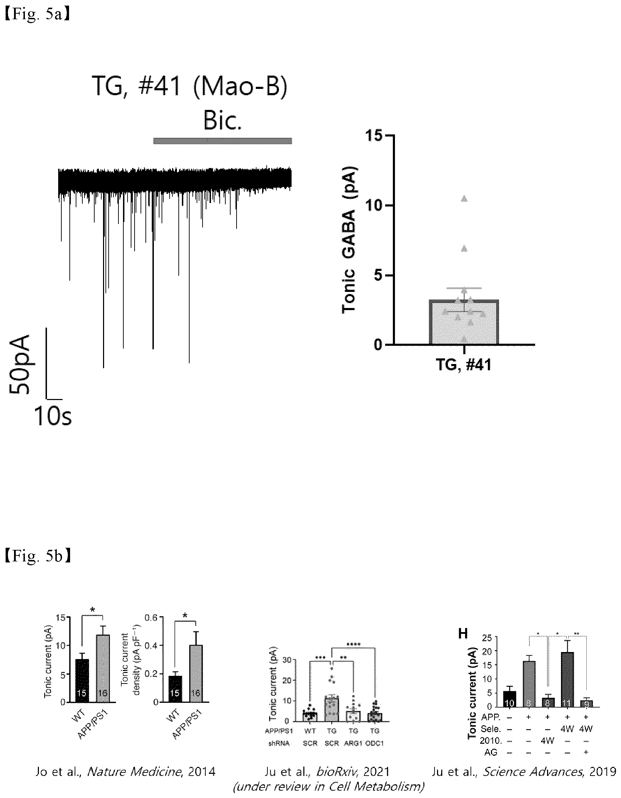

FIG. 5a shows the results of the experiment performed to measure the level of tonic GABA after injection of MAOB ASO into the Alzheimer's model. As a result of injecting MAOB ASO a41 into the Alzheimer's disease animal model APP/PS1, tonic GABA at a level of approximately 3.2 pA was measured. This is a relatively low amount compared to the levels of tonic GABA previously measured in the same Alzheimer's disease animal model as shown in FIG. 5b.

### 3-4. MAOB ASO KD Test (Measurement of Body Weight Change) in High-Fat-Diet Mice

All experiments performed in the high-fat diet mouse model were performed using mice obtained in a C57BL/6J background from the Jackson Laboratory (Stock number 000664, USA). Six-week-old male C57BL/6J mice (DBL, Chungcheongbukdo, Republic of Korea) were fed a high-fat diet (60% kcal fat, D12492, Research Diets Inc.) or a normal diet (Teklad, 2018S, Envigo) for 6 to 23 weeks. PBS and 3.5 mg/kg of ASO (a143) were used as controls, and MAOB-targeting ASOs (a41 and GalNAc-conjugated a41) were used. High-fat diet mice were anesthetized with vaporized isoflurane, and 200 µl of ASO (3.5 mg/kg concentration) was injected subcutaneously into each mouse using a syringe while holding the nape of the neck. Two injections were given at weeks 0 and 2, respectively, during a total of 5 weeks of observation, and body weight was measured once a week from weeks 0 to 5.

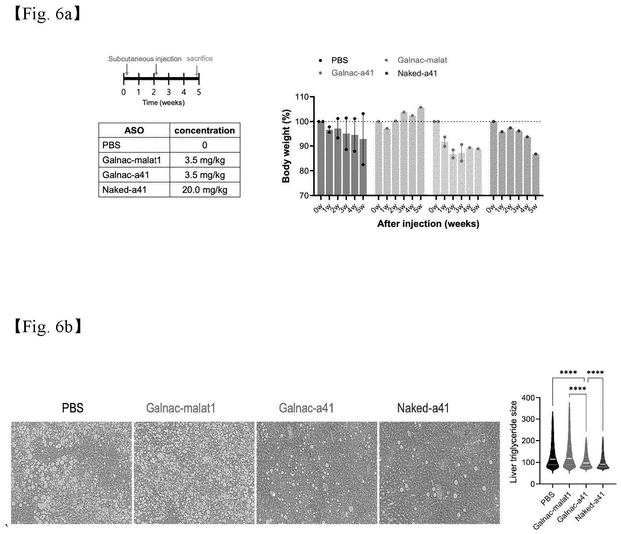

FIG. 6a shows the results of the experiment performed to evaluate the weight loss effect after injection of MAOB ASO into the fatty liver and obesity animal model. As a result of injecting MAOB-targeting ASO into the high-fat diet mice as the fatty liver and obesity animal model, it was confirmed that the body weight was significantly reduced compared to that of the control group.

### 3-5. MAOB ASO KD Test (Fatty Liver Phenotype) in High-Fat Diet (HFD) Mice

The mice used in Example 3-4 were deeply anesthetized with isoflurane at week 5, and then immediately their organs were isolated. Liver tissues were fixed with 4% PFA overnight, and further processed. Histological changes of lipid droplets were examined by hematoxylin and eosin (H&E) staining. As a counterstain, Mayer's hematoxylin was used for every slide.

A significant increase in triglyceride levels was observed in the livers of the high-fat diet mice, which is a trait similar to that of fatty liver. FIG. 6b shows the results of the experiment on the effect of MAOB ASO injection on recovery from fatty liver in the high-fat diet mice. As a result of injecting MAOB-targeting ASO into the high-fat diet mice, it was confirmed that the triglyceride level was significantly reduced compared to that in the control group.

### Industrial Applicability

According to the present invention, the antisense oligonucleotide may be effectively used for the prevention, alleviation or treatment of neurological disease or liver disease by reducing the amount of an mRNA encoding monoamine oxidase B or a protein encoded by the mRNA.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

### Sequence Listing Free Text

Electronic file attached.

## Claims

1. An oligomer of 13 to 35 nt in length, which inhibits MAOB gene expression by hybridization with at least 13 contiguous nucleobases of a whole pre-mRNA sequence of MAOB set forth in SEQ ID NO: 41 through A:T or G:C Watson-Crick pairing or G:U, I:A, I:C, or I:U wobble pairing.

2. The oligomer according to claim 1, wherein the oligomer is capable of hybridizing with at least 13 contiguous nucleobases of a sequence selected from the group consisting of SEQ ID NOs: 1 to 20 through A:T or G:C Watson-Crick pairing or G:U, I:A, I:C, or I:U wobble pairing, and is 13 to 35 nt in length.

3. The oligomer according to claim 1, wherein the oligomer is capable of hybridizing with at least 13 contiguous nucleobases of a sequence selected from the group consisting of SEQ ID NOs: 2, 3, and 9 through A:T or G:C Watson-Crick pairing or G:U, I:A, I:C, or I:U wobble pairing, and is 13 to 35 nt in length.

4. The oligomer according to claim 1, wherein the oligomer is capable of hybridizing with at least 13 contiguous nucleobases of the sequence SEQ ID NO: 9 through A:T or G:C Watson-Crick pairing or G:U, I:A, I:C, or I:U wobble pairing, and is 13 to 35 nt in length.

5. The oligomer according to claim 1, wherein the oligomer has the following sequence and chemical structure:
T*GAAC*6*6*5*5*5*7*7*6*5*6*ACGA*G;
G*ATCA*6*5*7*7*6*6*8*6*8*6*CAGC*T; or
C*ACTA*5*8*6*5*6*5*8*5*8*8*TAGC*C,
wherein PS represents phosphorothioate, and 2'MOE represents 2'-O-methoxyethyl, A=2'MOE-A, C=2'MOE-5'-methyl-C, G=2'MOE-G, T=2'MOE-T, 5=DNA-A, 6=DNA-5'-methyl-C, 7=DNA-G, 8=DNA-T, and *=PS.

6. The oligomer according to claim 1, wherein the oligomer has the following sequence and chemical structure:
C*ACTA*5*8*6*5*6*5*8*5*8*8*TAGC*C,
wherein PS represents phosphorothioate, and 2'MOE represents 2'-O-methoxyethyl, A=2'MOE-A, C=2'MOE-5'-methyl-C, G=2'MOE-G, T=2'MOE-T, 5=DNA-A, 6=DNA-5'-methyl-C, 7=DNA-G, 8=DNA-T, and *=PS.

7. The oligomer according to claim 6, wherein the oligomer comprises N-acetylgalactosamine (GalNAc).

8. The oligomer according to claim 7, wherein trivalent N-acetylgalactosamine (GalNAc) is linked to a 5' or 3' terminal phosphate of the oligomer.

9. A composition comprising a salt of the oligomer compound according to any one of claims 1 to 8 and at least one pharmaceutically acceptable carrier or diluent.

10. The composition according to claim 9, wherein the salt is a sodium salt or a potassium salt.

11. The composition according to claim 9, which is for preventing, alleviating or treating a neurological disease.

12. The composition according to claim 11, wherein the neurological disease is Alzheimer's disease.

13. The composition according to claim 9, which is for preventing, alleviating or treating a liver disease.

14. The composition according to claim 13, wherein the liver disease is fatty liver.

15. The composition according to claim 9, which is for preventing, alleviating or treating a metabolic disease.

16. The composition according to claim 15, wherein the metabolic disease is obesity.
[pre-mRNA sequence of MAOB]
SEQ ID NO. 41: chrX:43,766,610-43,882,450 (hg38, (-) strand, length 115,841 bp)
[Target and ASO sequences]
